# EUROPEAN PATENT APPLICATION

(11) **EP 2 070 471 A1**
(43) Date of publication of application: **17.06.2009**
(21) Application number: 07730475.6
(22) Date of filing: 26.04.2007
(51) Int. Cl.: A61B 5/0205

(54) **SYSTEM FOR MONITORING AND ANALYSING CARDIORESPIRATORY SIGNALS AND SNORING**

(30) Priority: 27.09.2006 ES 200602453
(71) Applicant: Universidad de Cadiz, 11001 Cadiz (ES)
(72) Inventor: ROJAS OJEDA, Juan Luis, 11002 Cádiz (ES); LEÓN JIMÉNEZ, Antonio, 11003 Cádiz (ES); CRESPO FOIX, Luis Felipe, 11002 Cádiz (ES); GROSS, Nicole, 11002 Cádiz (ES); SANCHEZ MORILLO, Daniel, 11002 Cádiz (ES)
(74) Representative: ABG Patentes, S.L.
(86) International application number: PCT/ES2007/000254
(87) International publication number: WO 2008/037820

(57) **Abstract**

The system object of this patent is based on the extraction of components of the signal captured by an accelerometer, obtaining information about physiological data such as cardiac, respiratory and snoring activity.

This system:
- provides information of the different cardiorespiratory variables useful for the diagnosis of the different types of abnormal respiratory phenomena during sleep (apneas, hypopneas and respiratory efforts associated to micro-arousals)
- assists with the diagnosis of respiratory disorders evaluated over long periods of time
- allows the verification and subsequent assessment of the appropriate airway pressures of continuous positive airway pressure (CPAP) equipment and bi-level positive airway pressure (BIPAP) equipment by means of detecting abnormal respiratory phenomena during the application of these therapies.

## Description

### Technical Field of the Invention

The system object of this patent is based on the extraction of components of the signal captured by an accelerometer, obtaining and processing information about physiological data. It is therefore an invention linked to the field of bioengineering, with applications in the field of medicine, allowing monitoring various biomedical parameters and assisting with the diagnosis of the sleep apnea-hypopnea syndrome (SAHS) and of other cardiorespiratory disorders.

### State of the Art and Background of the Invention

Within the various groups established by the International Classification of Sleeping Disorders (ICSD), the sleep apnea-hypopnea syndrome (SAHS) is included in the first group "Intrinsic Sleep Disorders". SAHS is produced by the intermittent and repetitive obstruction of the upper airway during sleep, which causes a complete (apnea) or partial (hypopnea) interruption of the air flow, being presented with **symptoms characterized by drowsiness, secondary cardiorespiratory and neuropsychiatric disorders,** SaO₂ reductions and transient arousals giving rise to a non-restorative sleep [1],[2].

The diagnosis of SAHS is currently not easy. On one hand, the clinical suspicion of the primary care physician is involved, who on many occasions has problems in referring the patient to a pneumology service for his or her study, diagnosis and treatment. This entire process is usually long and requires complex and expensive diagnostic studies.

The de facto standard for the diagnosis of these pathologies is polysomnography (PSG), which by means of studying the patient at night, records various body functions during sleep, such as the electrical activity of the brain, eye movement, muscular activity, pulse, respiratory effort, air flow and oxygen concentration in the blood. This requires connecting several sensors to the body of the patient: head, neck, arms and legs, ... and the subsequent manual analysis of the obtained record by specialists for the purpose of determining whether or not the disorder exists [3].

PSG requires, on most occasions, staying in a sleep unit (for one night in which 1 specialist technician is present) for which there are considerable waiting lists. This makes that a considerable number of cases with moderate-severe apnea remain undiagnosed. This problem leads to the increasing interest in finding alternative approaches for diagnosis, such as portable methods.

The use of alternative methods to PSG for evaluating patients who can suffer from sleep apnea has been the cause of multiple reviews in the literature. [4],[5],[6]

In all the reviews, portable monitoring systems, classified by the American Sleep Disorders Association, are based on using multiple sensors for monitoring and recording physiological signals, basic parameters such as oxygen saturation in the blood and/or oronasal flow, but their use is not very widespread due to their multiple obstacles.

Yoshiro Nagai and Kitajima Kazumi [12] propose, together with pulse oximetry measurements, the use of a three-axis acceleration sensor for determining the position of the patient in the bed for the purpose of correlating it with the oximetry measurement, supposedly to be able to determine the AHI indexes (apnea and hypopnea indexes). This system and other similar ones do not provide data about the snoring activity or about the heart rate.

The methods commonly used to record the cardiac, respiratory and snoring signal in respiratory polygraphies are described below.

The oronasal flow is experimentally quantified in an optimal manner by means of a pneumotachograph but this method is never in portable systems because it requires a mask strongly adhered to the face of the patient, potentially capable of interfering with sleep by itself.

Bucconasal thermistors are traditionally used for detecting respiratory events (recording temperature changes as a reflection of the air flow). The results obtained with these systems are good in the diagnosis of apneas but show limitations in the detection of hypopneas.

The use of a standard nasal cannula housed in the nasal cavities is currently in practice, which cannula is connected to a transducer detecting pressure changes conditioned by inspiration and expiration. It is an alternative system for the diagnosis of subtler respiratory events, providing a quantitative flow signal which does not require a nasal mask as in the case of pneumotachography. [7],[8],[9].

John G. Sotos et al. [13] use the signal of a microphone to evaluate aspects related to breathing while the patient is awake or asleep, without this information providing relevant data with respect to suffering from the apnea syndrome.

John G. Sotos et al [14], by means of two 2-axis acceleration sensors, capture the tracheal vibrations related to the breathing and the position of the subject, which factors may be necessary for defining some respiratory deficiencies but are not fundamental in the diagnosis of the different types of apnea.

Silva et al. [15] propose a system based on a microprocessor system with an accelerometer for studying breathing in animals and the sudden infant death syndrome (SIDS).

David Francois [17] proposes the use of a microphone located on the neck of the patient to detect hypoventilation states, establishing a non-detailed correlation of the latter with the apnea and hypopnea indexes. Rymut et al. [18] use a piezoelectric sensor designed by them located on the neck to determine several respiratory conditions of the patient, based on recording acoustic vibrations in the throat of such patient. Schechter et al. [19] use an accelerometer on the neck of the patient to record acoustic vibrations, which are compared with breathing patterns, to identify several disorders.

The non-respiratory parameter studied by the system is the electrocardiogram. Electrocardiography (ECG or EKG), representing the electrical activity of heart cells, is used as a standardized method for recording the cardiac signal. For its application, electrodes are fixed on the chest, for which it is occasionally necessary to clean the area, shave or move the hair out of the way. On other occasions, the cardiac signal is monitored by means of the pulse wave of the pulse oximeter.

Sierra, Gilberto et al. [10] describe a method and device for the non-invasive monitoring of the respiratory rate, heart rate and apnea by means of a sensor detecting the sounds and biological vibrations coming from the throat, displaying on a screen the results of the applied algorithms. This method and all those based exclusively on the sound recording technique have the drawback of their application in non-snoring patients who can present obstructive and/or central apneas. In addition, the heart rate obtained through recording tracheal sounds is subject to multiple artifacts due to the calculation algorithms used in this application (derived from the use of signals in the 20-200 Hz band).

Neil Townsend and Stephen Collins [11] describe a system for displaying heart and respiratory rate based on the spectral analysis of the signal from one or several accelerometers. This system only provides these two parameters using one-axis and/or two-axis sensors, and by means of autoregressive (AR) or FFT type algorithms. This information is very limited especially if it is aimed at the diagnosis of sleep apnea, given that the spectral analysis only provides mean values in a broad signal range. However, the continuous monitoring of the respiratory signal allows detecting events such as the cessation or resumption of chest movements, as well as the change in their intensity, typical in patients with sleep apnea, and the continuous monitoring of which is enabled by means of the system proposed in the present invention.

Finally, with respect to snoring, it is recorded in practice by a small, generally piezoelectric microphone located in the pretracheal area.

Campos et al. [16] propose a system including the hardware necessary for analyzing tracheal snores by means of placing a high-sensitivity microphone for recording the sounds. This information aimed at pathologies related to apnea is insufficient for a moderately efficient diagnosis.

### Literature

[1] H. Peter, T. Podszus, and P. von Wichert, Sleep Related Disorders and Internal Diseases. New York: Springer-Verlag, 1987, pp. 101-107.
[2] American Sleep Disorders Association Task Force, "The Chicago criteria for measurements, definitions, and severity of sleep related breathing disorders in adults," in Assoc. Professional Sleep Soc. Conf., New Orleans, THE, 1998.
[3] C. Guilleminault and M. Partinen, Obstructive Sleep Apnea Syndrome, Clinical Diagnosis & Treatment. New York: Raven, 1990.
[4] Ross SD, Allen IE, Harrison KJ, et al. Systematic review of the literature regarding the diagnosis of sleep apnea: evidence report/technology assessment No. 1. Rockville, MD: Agency for Health Care Policy and Research; February 1999; AHCPR Publication No. 99-002
[5] Flemons W, Littner M, Rowley J, et al. Home diagnosis of sleep apnea: a systematic review of the literature; an evidence review cosponsored by the American Academy of Sleep Medicine, the American College of Chest Physicians, and the American Thoracic Society. Chest 2003; 124:1543-1579.
[6] Nancy A. Collop. Portable Monitoring for Diagnosing Obstructive Sleep Apnea: Not Yet Ready for Primetime. Chest 2004; 125; 809-811
[7] Norman R, Ahmed M, Walsleben J, et al. Detection of respiratory events during NPSG: nasal cannula/pressure sensor versus thermistor. Sleep 1997; 20: 1175-1184.
[8] Montserrat J, Farre R, Ballester E, et al. Evaluation of nasal prongs for estimating nasal flow. Am J Respir Crit Care Med 1997; 155: 211-215.
[9] Hosselet J, Norman R, Ayappa I, et al. Detection of flow limitation with a nasal cannula/pressure transducer system. Am J Respir Crit Care Med 1998; 157: 1461-1467.
[10] Gilberto S, Victor L, et al. Non invasive monitoring of respiratory rate, heart rate and apnea. International application published under the patent cooperation treaty (PCT). WO 2005/096931 A1, 2005.
[11] Neil T, Stephen C. Respiration and heart rate monitor. International application published under the patent cooperation treaty (PCT). WO 03/005893 A2, 2003.
[12] Yoshiro Nagai, Kitajima Kazumi. Sleep evaluation method, sleep evaluation system, operation program for sleep evaluation system, pulse oximeter, and sleep support system. US Patent application publication 2006/0173257 A1, 2006.
[13] John G. Sotos et al. System and method for assessing breathing. US Patent application publication 2006/0155205 A1, 2006.
[14] John G. Sotos et al. Method and apparatus for evaluation of sleep disorders. US Patent application publication 2005/0113646 A1, 2006.
[15] Silva et al. Monitoring respiratory device. International application published under the patent cooperation treaty (PCT), WO 2004/043263 A2, 2004.
[16] Campos et al. Procedure for analysis of snoring and apnea and apparatus to carry out this analysis. European Patent Application, EP 1 410 759 A1, 2004.
[17] David Francois. Dispositif pour surveiller la respiration d'un patient. Institut national de la propieté industrielle - PARIS. Publication 2847796/ 02 14920, 2004.
[18] Rymut et al. Method and apparatus for monitoring respiration. US Patent application publication 2002/0072685 A1, 2002.
[19] Schechter et al. Graphical readout of laryngotracheal spectra and airway monitor. US Patent. Patent number 5,058,600, 1991.

### Description of the Invention

The object of the invention consists of a one-sensor system with high sensitivity and sufficient bandwidth to allow capturing physiological signals (cardiac, respiratory and snoring signal) from which fundamental parameters for monitoring and assisting with the diagnosis of SAHS and other cardiorespiratory pathologies are extracted.

The system object of this patent provides a simple and reliable alternative to current diagnosis methods allowing their application at home by unskilled people.

The system set forth fundamentally provides information of the different cardiorespiratory variables useful for the diagnosis of the different types of abnormal respiratory phenomena during sleep (apneas, hypopneas and respiratory efforts associated to micro-arousals, respiratory and heart rate disorders), recording scalar one-dimensional temporal series of these physiological variables, and treating them by means of different digital signal processing techniques.

The invention allows:
- Integration of the current equipment for monitoring and recording cardiac data, chest respiratory movements and snoring in a one-sensor system in a novel manner, using a single acceleration sensor, as a substitution for the electrodes used for the cardiac recording, the thermistor or cannula used for recording the respiratory flow, chest and abdominal respiratory movement detection bands and the microphone used for recording snoring.
- Processing of the captured data, by means of a microprocessor-based system, to continuously extract and monitor over time the mentioned physiological variables: sonocardiogram (SCG), thoracic respirogram (TRG) and snoring and whistling sounds (SWS).
- Display of parameters resulting from the analysis of the previous variables: heart rate (HR), heart rate variability (HRV), sympathetic-parasympathetic activity (SPA), brady-tachypnea (BTA), vagal sensor activity, snoring activity: events/hour.
- The application at home by storing or transmitting the data for its interpretation by a specialist.

### Brief Description of the Drawings

To make the object of the invention more intelligible, it has been illustrated with three schematic figures, which have the nature of a demonstrative example:
Figure 1 is an orientating view of the location of the sensor in practice. The three-axis accelerometer sensor must be fixed to the surface of the skin, on the suprasternal notch (1) and its output must be connected to the signal acquisition and treatment microprocessor system.
Figure 2 schematically depicts the physical system on which the invention is based and Figure 3 shows the different information processing steps until the generation of the information useful for the diagnosis.
Accompanying these 3 figures, there are another eight figures attached which graphically show the results expressed herein and which allow reliably comparing the information expressed in the description of the invention below.

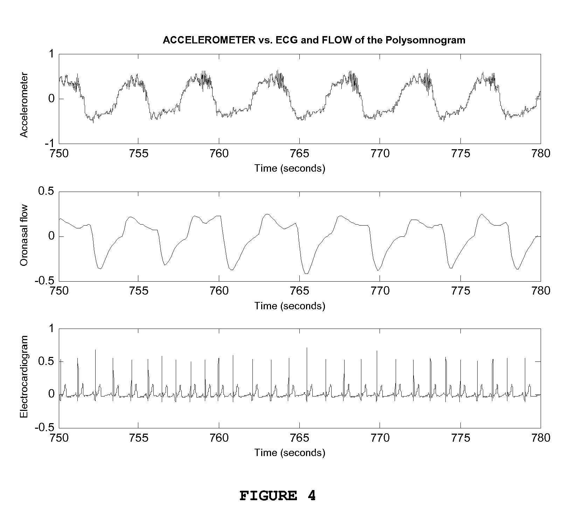

Figure 4 shows the form of the data captured by the accelerometer and the corresponding records of the ECG and flow signals captured by the conventional sensors used in nocturnal polysomnography.
Figure 5 shows the accelerometer signal and the component of the cardiac signal extracted in the same time interval by means of filtering.
Figures 6 and 7 show the results provided by the algorithms applied to that of the obtained cardiac signal.
Figure 8 shows the extracted respiratory component and the oronasal flow measurement obtained by means of thermistors included in the PSG. Both signals are superimposed in the lower part, a perfect correspondence being verified.
Figures 9 show the oxygen saturation (SpO₂) and respiratory flow signals provided by the polysomnogram (PSG) and the corresponding filtered accelerometer signal during a typical episode of obstructive apnea.
Figure 10 shows the results of the process for extracting high-frequency components from the accelerometer signal linked to the emitted sounds, and especially to snoring.

### Detailed Description of the Invention

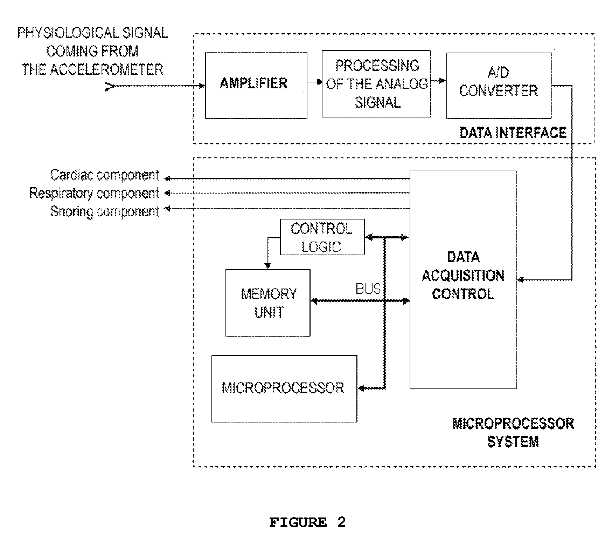

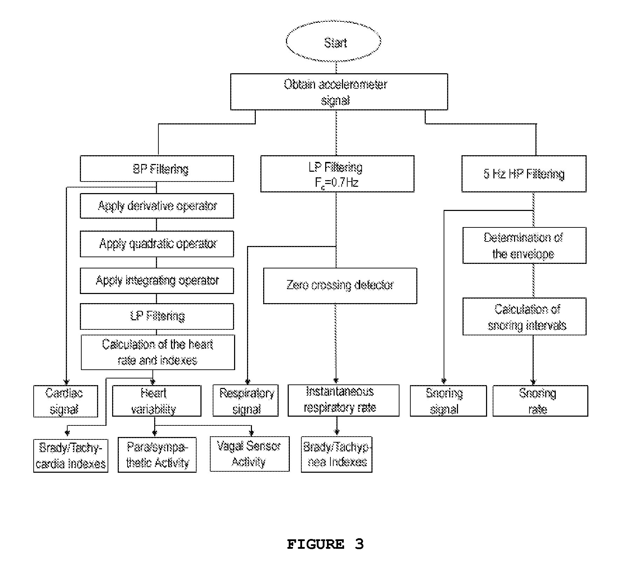

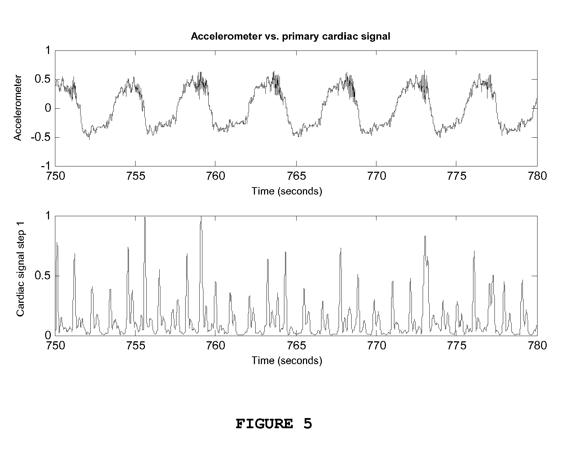

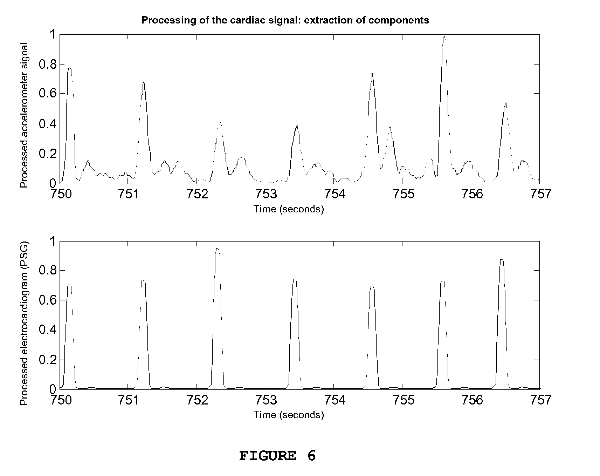

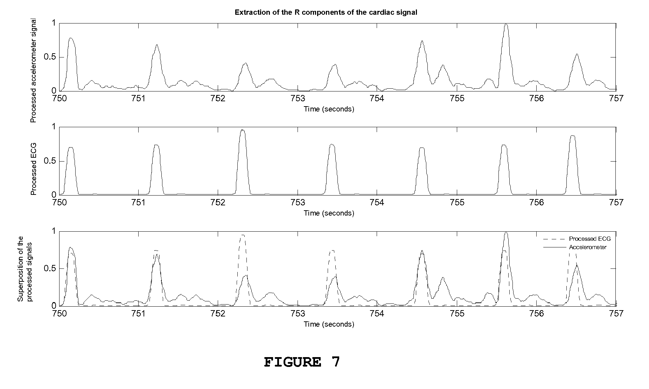

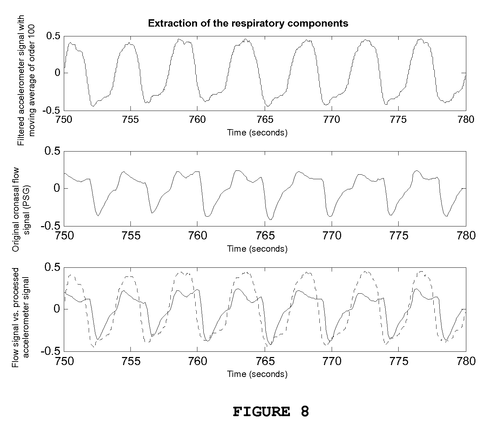

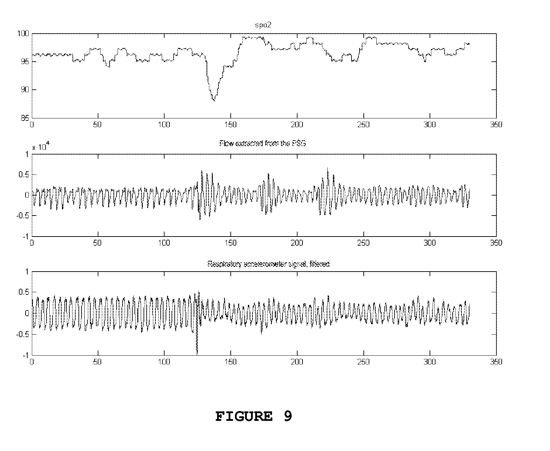

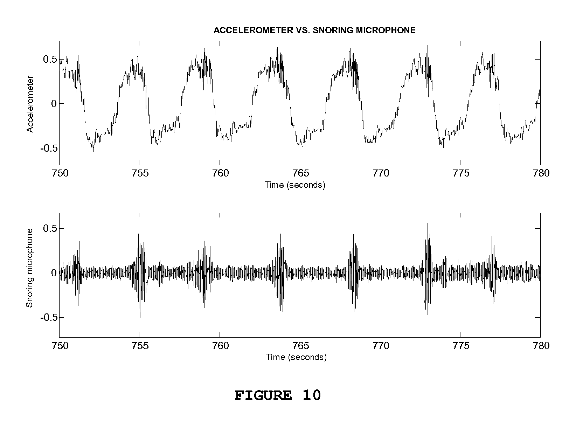

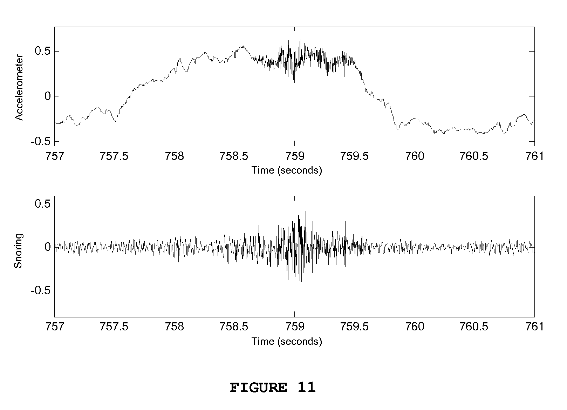

The system comprises the following physical and logic (hardware and software) components:
1. An accelerometer with a sensitivity equal to or greater than 100 mV/g and a frequency response of ± 3 dB between 0.1 and 2000 Hz. It is important to emphasize that an accelerometer records the components of the acceleration on its sensitive axes. It is possible to find different two-axis or three-axis sensors meeting the mentioned specifications, and any of them can be used for the invention set forth. The accelerometer signal is conditioned in a preprocessing unit by means of a preamplifier, amplifier and anti-aliasing filter.
2. The microprocessor-based system controls the sampling to frequencies not less than 1024 samples with a resolution of 12 to 16 bits. This microprocessor system can be physically implemented by one or several devices, capable of fulfilling the described functions. They can be general or specific purpose systems such as microprocessors, microcontrollers, digital signal processors, application-specific integrated circuits (ASICs), personal computers, PDAs, smartphones, etc.
3. The data will be stored in any storage system or combination thereof, such as volatile memories (DRAM), non-volatile memories, hard drives, CD-RW, DVD, removable memories (SD, MMC cards, ...) with a capacity equal to or greater than 500 Mbytes. (See Figure 2).
4. A prior filtering of the previous work space is applied to eliminate artifacts in the measurement, generating a new record of fault-free data. This preprocessing can include the truncation or the interpolation on the original record, and the standardization of the set of data, contemplating the elimination of data above a certain threshold (Figures 2 and 3).
5. According to the process indicated in Figure 3, it extracts the cardiac, respiratory and snoring components. The independent triple processing of the signal allows extracting the cardiac (Figures 5, 6 and 7), respiratory (Figures 8 and 9) and snoring (Figures 10 and 11) variables contained in the captured signal. In the case of the cardiac signal, the analysis is focused on the [0.5, 3 Hz] frequencies and for the respiratory signal, the [0, 0.5 Hz] frequencies are studied. Snoring is studied in the rest of the upper spectrum of the signal.
6. For the extraction of the respiratory component a prior low-pass filtering is performed with a cutoff frequency of about 0.7 Hz, since the respiratory functions fluctuate below this limit. The signal at the exit of this filtering step has a high correlation with the signal corresponding to the oronasal flow captured with a thermistor, as can be seen in Figure 8. The calculation of the respiratory rate is shown in Figure 8. The signal resulting from the previous low-pass filtering is subjected to a calculation step in the time domain, based on a zero crossing estimation algorithm. The instantaneous respiratory rate is obtained directly from this value. The different types of respiration rates (normal respiration, tachypnea and bradypnea) are derived from these values. The respiratory component and the rate are stored by the system.
7. The R peaks, caused by the ventricular concentration and corresponding to the QRS complex of the cardiac signal and the intervals therebetween (RR interval), which allow studying the heart rate variability (HRV), are extracted by means of the processing detailed in Figure 3. This processing is based on the commonest algorithms for detecting QRS complexes, although other algorithms leading to efficient results in the detection of the R peaks can be used (Figures 5, 6 and 7).
8. Finally, the component corresponding to snoring can be extracted from the electrical signal at the output of the acceleration sensor, applying a band-pass filter with cutoff frequencies of about the voice frequencies. Figures 10 and 11 show the signals coming from the acceleration sensor and from a microphone captured for a patient. The snoring intervals are identified at the exit of the filtering step. These intervals can aid in identifying segments of interest for a more specific analysis of the other components. The snoring signal at the exit of the filter and the corresponding intervals are stored for a possible subsequent evaluation.

### Advantages Provided by the Invention

In relation to the invention set forth, a detailed method is provided for identifying possible respiratory disorders, such as the sleep apnea-hypopnea syndrome (SAHS), respiratory, cardiac, cardiorespiratory, pneumological diseases or the like, or sudden infant death syndrome.

The invention described and set forth consequently involves a simplification of the tests for the diagnosis of certain dysfunctions associated to sleep disorders such as the sleep apnea-hypopnea syndrome (SAHS), provides an aid for the diagnosis of cardiorespiratory disorders evaluated over long periods of time and furthermore has applications beyond respiratory physiology (e.g., cardiology).

The following are emphasized among the advantages provided with respect to the current state of the art:
1. System with a simple application and operation.
2. It does not require skilled personnel.
3. Use at home and use in hospital.
4. Use in disaster and emergency situations for the quick discrimination of the vital situation of the affected people.
5. Integration of the 3 currently used systems in a single sensor.
6. Novel processing of the captured information for continuously obtaining and monitoring three biological signals useful for the diagnosis, as well as different rates and indexes derived therefrom.

### Embodiment of the Invention

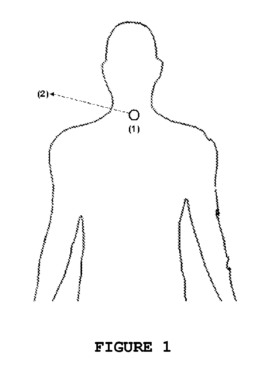

The proposed method includes the following phases:
1. Test for collecting data from the patient, with the placement of the two- or three-axis accelerometer sensor fixed to the surface of the skin, on the suprasternal notch (Figure 1). The test is performed for a period preestablished by the specialist and allows generating a first work space. It is aimed for its performance at night and allows records of up to 10 hours.
2. The data is acquired by the acquisition system which conditions the signal by means of a preamplifier, amplifier and anti-aliasing filter. The sampling is done with frequencies not less than 1024 samples. The obtained data is stored in a record for its processing or transmission.
3. A prior filtering of the signal is applied to eliminate artifacts in the measurement, generating a new record of fault-free data. This preprocessing can include the truncation or the interpolation on the original record, and the standardization of the set of data, contemplating the elimination of data above a certain threshold (Figures
   2 and 3) .
4. Independent triple processing in the time domain to extract the cardiac (Figures 5, 6 and 7), respiratory (Figures 8 and 9) and snoring (Figures 10 and 11) components contained in the captured signal.
5. Delivery of the result of the processing to a decision-making step for generating the output information, containing useful guidelines for the specialist to facilitate the diagnosis. The results are determined immediately and can be presented to the patient by his or her specialist doctor as soon as the test ends (Figure 2).

## Claims

1. A system for monitoring and analyzing cardiorespiratory and snoring signals, **characterized by** using a single acceleration sensor for continuously capturing and monitoring physiological signals in the time domain (cardiac, respiratory and snoring signal), from which other fundamental parameters are extracted to assist with the diagnosis of the sleep apnea-hypopnea syndrome and of other cardiorespiratory disorders.

2. The system for monitoring and analyzing cardiorespiratory and snoring signals according to claim 1, comprising:
a. An accelerometer for recording on its sensitive axes the acceleration components of the cardiac, respiratory and snoring components.
b. A data acquisition system for conditioning the signal by means of a preamplifier, amplifier and anti-aliasing filter and an analog-to-digital converter.
c. A microprocessor-based system, by means of which the triple processing of the signal will be performed, from which signal the cardiac, respiratory and snoring variables contained therein will be extracted by means of filtering and temporal treatment techniques and will be displayed to allow the diagnosis of the specialist.
d. System for storage and transmission of the previous data.

3. A process for monitoring and analyzing cardiorespiratory and snoring signals, using the system described in claims 1 and 2, comprising:
a) Test for collecting data from the patient, performed by means of placing the accelerometer sensor fixed to the surface of the skin on the suprasternal notch.
b) Filtering the obtained data to eliminate artifacts in the measurement, standardizing and generating a new record of fault-free data.
c) Triple processing of the signal to extract the cardiac, respiratory and snoring variables contained in the captured signal.
d) Display of the useful output information to the specialist to facilitate the diagnosis.
e) Analysis of the displayed information, which must be interpreted by the specialist doctor.

4. The process for monitoring and analyzing cardiorespiratory and snoring signals according to claim 3, **characterized in that** the analysis of the cardiac signal is focused on the [0.5, 3 Hz] frequencies, the analysis of the respiratory signal is focused on the [0, 0.5 Hz] frequencies and the snoring is studied in the rest of the upper spectrum of the signal.

5. The process for monitoring and analyzing cardiorespiratory and snoring signals according to claims 3 and 4, **characterized in that** for the extraction of the respiratory component a prior low-pass filtering is performed with a cutoff frequency of about 0.7 Hz, which allows its continuous monitoring.

6. The process for monitoring and analyzing cardiorespiratory and snoring signals according to claims 3 to 5, **characterized in that** the signal resulting from the low-pass filtering is subjected to a calculation step in the time domain, based on zero crossing detection, which allows directly obtaining the respiratory rate from this value.

7. The process for monitoring and analyzing cardiorespiratory and snoring signals according to claims 3 and 4, **characterized in that** the extraction of the cardiac component, by means of an algorithm in the time domain, is performed in the following steps:
a. Applying a derivative operator.
b. Obtaining absolute values by means of a quadratic operator.
c. Integrating data obtained in the previous step.
d. Low-pass filtering to eliminate noises.
e. Calculating and averaging the intervals between heartbeats.

8. The process for monitoring and analyzing cardiorespiratory and snoring signals according to claims 3, 4 and 7, **characterized in that** the R peaks of the cardiac signal and the corresponding RR intervals, the heart rate and variability are extracted by means of the processing detailed in claim 7.

9. The process for monitoring and analyzing cardiorespiratory and snoring signals according to claims 3, 4, 7 and 8, **characterized in that** starting from the data obtained in the heart variability, other data reflecting the sympathetic, parasympathetic and baroreflex sensor activity is obtained.

10. The process for monitoring and analyzing cardiorespiratory and snoring signals according to claims 3 and 4, **characterized in that** the component corresponding to snoring is extracted from the electrical signal at the output of the acceleration sensor, applying a band-pass filter with cutoff frequencies of about the voice frequencies.

11. The process for monitoring and analyzing cardiorespiratory and snoring signals according to claims 3, 4 and 10, **characterized in that** the snoring intervals are identified at the exit of the filtering step, which intervals aid in identifying segments of interest for a more specific analysis of the other components.

12. A domestic use of the system and process for monitoring and analyzing cardiorespiratory and snoring signals according to claims 1 to 11 for detecting cardiorespiratory events.

13. A domestic use of the system and process for monitoring and analyzing cardiorespiratory and snoring signals according to claims 1 to 11 for the supervision and monitoring at home.

14. A use of the system and process for monitoring and analyzing cardiorespiratory and snoring signals according to claims 1 to 11 for their use in hospital in surgery and intensive care units.

15. A use of the system and process for monitoring and analyzing cardiorespiratory and snoring signals according to claims 1 to 11 in disaster and emergency situations for the quick discrimination of the vital situation of the affected people based on the study of their cardiorespiratory physiological variables.
